# EUROPÄISCHE PATENTSCHRIFT

(11) **EP 2 346 434 B1**
(45) Veröffentlichungstag und Bekanntmachung des Hinweises auf die Patenterteilung: **15.05.2013**
(21) Anmeldenummer: 09777984.7
(22) Anmeldetag: 19.08.2009
(51) Int. Cl.: A61B 18/20, A61C 1/00

(54) **LASERBEARBEITUNGSGERÄT ZUR BEARBEITUNG VON BIOLOGISCHEM GEWEBE**
LASER PROCESSING DEVICE FOR PROCESSING BIOLOGICAL TISSUE
APPAREIL DE TRAITEMENT AU LASER DE TISSUS BIOLOGIQUES

(30) Priorität: 17.09.2008 DE 102008047640
(43) Veröffentlichungstag der Anmeldung: 27.07.2011
(73) Patentinhaber: Lumera Laser GmbH, 67661 Kaiserslautern (DE); Kasenbacher, Anton, 83278 Traunstein (DE)
(72) Erfinder: KASENBACHER, Anton, 83278 Traunstein (DE)
(74) Vertreter: Patentanwälte Lambsdorff & Lange
(86) Internationale Anmeldenummer: PCT/EP2009/006021
(87) Internationale Veröffentlichungsnummer: WO 2010/031478

(56) Entgegenhaltungen:
- WO-A-93/21992
- WO-A-2006/022970
- WO-A-2007/007336
- WO-A-2008/048832
- WO-A-2008/089292
- US-A1- 2004 259 053
- US-A1- 2006 195 072
- BRIAN C. WILSON, MICHAEL S. PATTERSON: "The physics, biophysics and technology of photodynamic therapy" PHYS. MED. BIOL., Bd. 53, 9. April 2008 (2008-04-09), Seiten R61-R109, XP002553539
- MARTIN E. FERMANN, ALMANTAS GALVANAUSKAS, GREGG SUCHA: "Ultrafast lasers: technology and applications; Volume 80 of Optical engineering (Marcel Dekker, Inc.)" 2003, CRC PRESS , XP002553542 Seite 343 - Seite 344
- TIMOTHY C. ZHU, JAROD C. FINLAY: "The role of photodynamic therapy (PDT) physics" MED. PHYS., Bd. 35, Nr. 7, 16. Juni 2008 (2008-06-16), Seiten 3127-3136, XP002553540
- K. KONOPKA, T. GOSLINSKI: "Photodynamic Therapy in Dentistry" JOURNAL OF DENTAL RESEARCH, Bd. 86, Nr. 8, 2007, Seiten 694-707, XP002553541

## Beschreibung

Die vorliegende Erfindung betrifft ein dentales Laserablationsgerät zur Ablation von Zahnmaterial gemäß dem unabhängigen Patentanspruch 1.

Das Anwendungsgebiet der vorliegenden Erfindung betrifft das Gebiet der Zahnheilkunde, wobei anstelle eines mechanischen Bohrers zur Ablation bzw. Abtragung von Zahnmaterial, insbesondere kariösem Zahnmaterial, ein Laserbearbeitungs-Verfahren und ein entsprechendes Laserbearbeitungsgerät zum Einsatz kommen können. Die Erfindung kann jedoch ebenso auf andere Arten von biologischem Geweben und Gewebetypen, wie beispielsweise Hartgewebe, Weichgewebe und Gewebeflüssigkeiten, angewendet werden.

In der Zahnheilkunde, insbesondere in der Kariestherapie, besteht ein wesentliches Ziel darin, die konventionelle Röntgentechnik für Diagnostik sowie die konventionelle mechanische Bohrervorrichtung ganz oder zumindest teilweise durch nahezu monochrome (LED) und/oder exakt monochrome Strahlungsquellen (Laser) zu ersetzen. Das mutagene und karzinogene Potenzial der Röntgenstrahlung ist hinlänglich in der Medizin bekannt, weshalb stets nach der grundlegenden Leitlinie des Strahlenschutzes, dem ALARA-Prinzip ("As Low As Reasonably Achievable"), gehandelt werden muss. Ideale Alternative wäre eine Analyse ohne Röntgenstrahlung, wenn möglich während der Behandlung. Was die Bearbeitung oraler Gewebsstrukturen anbelangt, so ist bis dato der konventionelle "Bohrer" wegen seiner Universalität bei geringen Investitionskosten trotz seines erheblichen thermo-mechanischen Schädigungspotenzials (Friktionswärme, Risse, Schockwellen) und daraus unvermeidbar resultierender Schmerzinduktion in der Zahnheilkunde das Mittel der Wahl. Ein "denkendes" Gerät für simultane und objektive Detektion pathologischer Strukturen (z.B. Karies) und Therapie (z.B. Kavitätenpräparation) mit automatischem selflimiting-Stopp bei maximaler Biosicherheit existiert bis dato weltweit nicht. Alle zuvor genannten erheblichen Nebenwirkungen sowie Nachteile lassen sich beim Einsatz eines kombinierten Diagnose- und Laserbearbeitungsgerätes vermeiden.

Im Laufe der letzten Jahre ist eine Reihe von Lasersystemen im Hinblick auf ihren Einsatz in der Zahnheilkunde untersucht worden. In vielen Fällen und gerade bei den ersten vorgeschlagenen Lasersystemen hat sich jedoch gezeigt, dass entweder unerwünschte thermische oder andere Nebeneffekte auftraten oder dass die Abtragungseffizienz sich als zu gering erwies. Dies gilt insbesondere für Lasersysteme auf der Basis von gepulsten Laserstrahlquellen, die mit Pulsbreiten im Bereich von Nano- bis Mikrosekunden arbeiten, wobei beispielsweise Excimer-Laser mit Wellenlängen im UV-Bereich oder Er: YSGG- (λ = 2,7 µm) oder Er:YAG-Laser (λ = 2,94 µm) im infraroten Wellenlängenbereich verwendet wurden. Keines dieser Systeme ist zudem in der Lage, eine biosichere Detektion und Therapie durchzuführen.

Ein wesentlicher Fortschritt ergab sich erst mit dem Einsatz von Kurzpuls-Lasersystemen im Pikosekunden- bzw. Femtosekundenbereich und Wellenlängen im sichtbaren bis nah-infraroten Spektralbereich. In ersten experimentellen Studien konnte bereits gezeigt werden, dass diese Systeme eine qualitativ hochwertige Zahnabtragung ermöglichen, wobei die Abtragungseffizienz zumindest vergleichbar ist mit der Leistungsfähigkeit einer mechanischen Turbine.

Die Druckschrift US 5,720,894 beschreibt ein Verfahren und eine Vorrichtung zum Abtragen von Material mittels einer gepulsten Laserstrahlquelle. Die zu wählenden Parameter bei der Abtragung hinsichtlich Wellenlänge, Pulsbreite, Energie und Wiederholrate der Laserpulse werden im Wesentlichen lediglich aufgabenhaft derart umschrieben, dass jeder Laserpuls mit einem dünnen Oberflächenbereich des Materials derart wechselwirken soll, dass im Fokus des Bearbeitungs-Laserstrahls ein Plasma gebildet wird. Für die genannten Parameter der gepulsten Laserstrahlung werden relativ weite Bereiche angegeben, wobei insbesondere hinsichtlich der Energie der Laserpulse angegeben wird, dass diese bis zu 50 mJ oder bezogen auf die Fläche bis zu 15 J/cm² betragen kann. Es ist jedoch zu befürchten, dass bei derartig hohen Pulsenergien bei sehr kurzen bis ultrakurzen Laserpulsen die Strahlungsleistung oder -intensität im Pulsmaximum Werte erreicht, bei denen durch nicht-lineare Prozesse wie Multiphotonen-Ionisation, insbesondere unter Beteiligung von mehr als drei Photonen, schädigende Nebeneffekte auftreten. Insbesondere wird bei derartigen Pulsspitzenleistungen (einige TW/cm²) das Wassermolekül (Ionisationsenergie Eᵢₒₙ = 6,5 eV) ionisiert mit fatalen Nebenwirkungen wie Schädigung der menschlichen Erbsubstanz (DNA) und Bildung sog. "Kavitationsbläschen" (im angloamerikanischen Sprachraum "cavitation bubbles" genannt) mit nachfolgenden unvermeidbaren Sonolumineszenzen bei einer spektralen Bandbreite vom UV (Ultraviolett)- bis in den Röntgenbereich hinein.

Der nächstliegende Stand der Technik der vorliegenden Erfindung ist in WO 2008/089292 A1 offenbart.

Es ist Aufgabe der vorliegenden Erfindung, ein dentales Laserablationsgerät zur Ablation von Zahnmaterial anzugeben, mit welchen eine effiziente Ablation des Zahnmaterials eines Gewebes gewährleistet werden kann und gleichzeitig eine Möglichkeit bereitzustellen, schädigende Einflüsse auf das bearbeitete Gewebe und die unmittelbare Umgebung zu vermeiden oder zu minimieren, und eine zusätzliche Flexibilität in der Wahl der Wellenlänge der Laserablation zu ermöglichen. Es ist insbesondere Aufgabe der vorliegenden Erfindung, ein Lasergerät zur Diagnose und Ablation von Zahnmaterial anzugeben, mit dem eine objektive Diagnose und effiziente Ablation eines Zahanmaterials sogar instantan gewährleistet und gleichzeitig eine Möglichkeit bereitgestellt wird, schädigende Einflüsse auf das diagnostizierte und bearbeitete Gewebe und die unmittelbare Umgebung zu vermeiden oder zu minimieren sowie eine zusätzliche Flexibilität in der Wahl der Laserwellenlänge, Laserpulsdauer und Laserintensität zu ermöglichen.

Diese Aufgabe wird mit den Merkmalen des Patentanspruchs 1 gelöst. Vorteilhafte Weiterbildungen und Ausgestaltungen sind Gegenstand von Unteransprüchen.

Eine Erkenntnis der vorliegenden Erfindung besteht darin, dass bei der Bearbeitung von biologischem Gewebe mit einem Laserstrahl nicht notwendigerweise das Gewebe selbst mit dem Laserstrahl beaufschlagt werden muss. Der Laserstrahl kann stattdessen in vorteilhafter Weise von einer Substanz absorbiert werden, welche infolge der Absorption im Wesentlichen als eine Quelle freier oder quasi freier Elektronen wirkt und solchermaßen die absorbierte Energie an das abzutragende Material überträgt. Als eine solche Substanz kann am wirkungsvollsten ein sogenannter Photosensitizer eingesetzt werden. Photosensitizer stellen chemische lichtempfindliche Verbindungen dar, die nach der Absorption eines Lichtquants eine fotochemische Reaktion eingehen. Die Aktivierung eines Photosensitizers kann durch Laserlicht geeigneter Wellenlänge und ausreichender Intensität erfolgen, wobei durch Lichtabsorption der Photosensitizer zuerst in einen relativ kurzlebigen Singulett-Zustand angeregt wird, der danach in einen stabileren Triplett-Zustand übergeht. Dieser angeregte Zustand kann dann beispielsweise direkt mit dem abzutragenden Material reagieren.

Eine weitere Erkenntnis der vorliegenden Erfindung besteht darin, dass Laserpulse mit einer zeitlichen Halbwertsbreite im Pikosekundenbereich vorteilhaft verwendet werden können. In diesem Bereich kann ein Optimum an Abtragungseffizienz einerseits sowie biologisch-medizinischer Verträglichkeit andererseits aufgrund der optischen Eindringtiefe und der dadurch möglichen Begrenzung thermischer und mechanischer Belastungen erreicht werden.

Eine weitere Erkenntnis der vorliegenden Erfindung besteht darin, für eine Diagnose, die gegebenenfalls simultan zu einem Bearbeitungsverfahren durchzuführen ist, einen Marker einzusetzen, der zu bearbeitende oder zu ablatierende Bereiche sichtbar macht, wobei der Marker ebenfalls ein Photosensitizer sein kann und/oder durch Strahlung eines Lasers oder einer Lichtemissionsdiode (LED) kontinuierlich oder gepulst, mit einer geeigneten Wellenlänge, Dauer und Intensität anregbar ist.

Eine weitere Erkenntnis der vorliegenden Erfindung besteht darin, einen mittels eines Laserstrahls zu bearbeitenden oder zu ablatierenden Bereich eines Gewebes mit einer Ummantelung zu umgeben und innerhalb dieser und einer damit verbundenen Laserstrahl-Auskoppeleinheit ein Absaugkanalsystem zu integrieren.

Beispielhaft, jedoch ohne einen Teil der Erfindung darzustellen, wird ein Verfahren zur Bearbeitung von biologischem Gewebe angegeben, bei welchem ein Photosensitizer in eine räumliche Umgebung eines zu bearbeitenden Bereiches des Gewebes gebracht wird, ein gepulster Bearbeitungs-Laserstrahl bereitgestellt wird, und der zu bearbeitende Bereich des Gewebes mit dem gepulsten Bearbeitungs-Laserstrahl bestrahlt wird, wobei der Bearbeitungs-Laserstrahl Laserpulse mit einer zeitlichen Halbwertsbreite in einem Bereich zwischen 1 ps und 100 ps emittiert, wobei dieser Bereich auch jeden inkrementellen Zwischenwert umfasst und das Inkrement 100 fs beträgt.

Gemäß eines weiteren Beispiels wird die Wellenlänge der Laserpulse derart eingestellt, dass der Bearbeitungs-Laserstrahl durch eine Zwei-Photonen-Absorption in dem Photosensitizer mindestens teilweise absorbiert wird, wobei die Absorption in der Nähe eines Absorptions-Maximums des Photosensitizers liegt.

Gemäß eines dazu alternativen Beispiels wird die Wellenlänge der Laserpulse derart eingestellt, dass der Bearbeitungs-Laserstrahl durch eine Ein-Photonen-Absorption in dem Photosensitizer mindestens teilweise absorbiert wird, wobei die Absorption in der Nähe eines Absorptions-Maximums des Photosensitizers liegt.

Gemäß eines Beispiels wird eine Wiederholrate der Laserpulse in einem Bereich zwischen 1 Hz und 1000 kHz eingestellt. Dabei kann auch vorgesehen sein, dass die Laserpulse in Form von Bursts mit jeweils einer vorgegebenen Anzahl von Laserpulsen erzeugt werden und beispielsweise jede zu bearbeitende Stelle auf dem zu ablatierenden Gewebe mit einer vorgegebenen Anzahl von Bursts (beispielsweise einem Burst) beaufschlagt wird. Innerhalb eines Bursts können die Laserpulse auch eine in vorgegebener Weise variierende Pulsspitzenintensität aufweisen. Vorteilhaft ist es, wenn vor, während oder nach den Bursts keinerlei unerwünschte Vor- oder Nachpulse oder Untergrund- und Offset-Intensitäten auftreten.

Gemäß eines Beispiels wird die Energiedichte der Laserpulse in einem Bereich zwischen 1,5 J/cm² und 7,5 J/cm² eingestellt. Insbesondere kann die Energie der Laserpulse in einem Bereich unterhalb von 100 µJ eingestellt werden und der Fokus des Bearbeitungs-Laserstrahls auf einer Oberfläche des Gewebes und/oder des Photosensitizers kann mit einem Fokusdurchmesser in einem Bereich zwischen 10 µm und 100 µm eingestellt werden.

Gemäß eines Beispiels liegt die Pulsspitzenintensität der Laserpulse bei der Bearbeitung in einem Bereich zwischen 10¹¹ W/cm² und 1,5 × 10¹² W/cm². Gemäß einer Ausführungsform liegt die Pulsspitzenintensität bei der Diagnose bei Verwendung gepulster Laserstrahlung in einem Bereich zwischen 10⁶ W/cm² und 10⁹ W/cm².

Gemäß eines Beispiels wird das Verfahren zur Ablation oder Abtragung von Zahnmaterial, insbesondere kariösem Zahnmaterial, eingesetzt. Dies bietet insofern ein vorteilhaftes Anwendungsgebiet, als kariöses Zahnmaterial bekanntermaßen eine durch den bakteriellen Befall bedingte poröse Struktur aufweist. In diese poröse Struktur kann der Photosensitizer eindringen, sodass er gewissermaßen in das abzutragende, kariöse Zahnmaterial eingebettet ist und nicht - wie in anderen Anwendungsfällen erforderlich - auf eine Oberfläche eines abzutragenden Gewebematerials aufgebracht werden muss.

Es ist bereits bekannt, dass bei der Bearbeitung von biologischem Gewebe mit einem Kurzpulslaser wie einem Piko- oder Femtosekundenlaser innerhalb einer dünnen Oberflächenschicht im Fokus des Bearbeitungs-Laserstrahls ein Mikroplasma erzeugt wird, welches nach jedem einzelnen Puls im Verlauf einer Zeitspanne im Nano- bis Mikrosekundenbereich zerfällt. Bei dem Ablationsvorgang wird das biologische Gewebe per Interaktion der Laserphotonen mit den quasi-freien Elektronen nicht ionisiert, sondern minimal-invasiv thermo-mechanisch fragmentiert. Ein generelles Ziel besteht darin, das Mikroplasma stets im Schwellenbereich zu generieren, d.h. stets unterhalb der kritischen Elektronendichte (bei LaserWellenlänge 1064 nm: 1,03 x 10²¹ Elektronen/cm³). Dadurch wird es möglich, den Ablationsvorgang unter größtmöglicher medizinischer und biologischer Verträglichkeit und Vermeidung unerwünschter Nebeneffekte durchzuführen. Insbesondere sollen Plasmatemperaturen von größer gleich 5800 K (Temperatur der Sonnenoberfläche) mit der Folge von UV-Strahlen sowie Multiphotonen-Ionisation vermieden werden, um so zu erreichen, dass in dem Gewebe vorhandene Wassermoleküle nicht ionisiert werden. Die erfindungsgemäße Kombination aus einem indirekten Energieeintrag mittels Photosensitizern und der Verwendung von Pikosekunden-Laserimpulsen gibt hier eine entscheidende Richtung für mehr biologisch-medizinische Verträglichkeit vor. Insbesondere hinsichtlich der Stressrelaxation wird durch die Pikosekunden-Laserpulse eine optische Penetrationstiefe derart bewirkt, dass sich keinerlei Schockwellen ausbreiten können und die Behandlung damit schmerzfrei durchgeführt werden kann.

Im Allgemeinen wird mit dem Bearbeitungs-Laserstrahl ein bestimmter Oberflächenbereich des biologischen Gewebes bearbeitet, sodass der Laserstrahl gemäß einer weiteren Ausführungsform in geeigneter Weise über diesen Oberflächenbereich gescannt oder gerastert wird. In diesem Zusammenhang kann es sich zusätzlich als vorteilhaft erweisen, wenn der Bearbeitungs-Laserstrahl ein im Wesentlichen rechteckförmiges Strahlprofil (im Englischen auch tophat) aufweist. Bei dem Scan-Vorgang kann dann vorgesehen sein, dass jeder von dem Fokus des Bearbeitungs-Laserstrahls erfasste Teilbereich von genau einem Laserpuls beaufschlagt wird. Diese Maßnahme kann auch unabhängig von dem Vorhandensein eines Tophat-Profils vorgesehen sein, etwa indem beispielsweise festgelegt wird, dass bei dem Scan-Vorgang einander benachbarte und von je einem Laserpuls erfasste Teilbereiche einen räumlichen Überlapp miteinander haben, dessen Fläche kleiner als die Hälfte oder kleiner als ein anderer Bruchteil der Fläche eines Teilbereichs ist. Auf diese Weise kann auch bei Vorhandensein eines Gauß-Profils als "Laserstrahl-Querschnitt" erreicht werden, dass im Wesentlichen ein von dem Fokus des Bearbeitungs-Laserstrahls erfasster Teilbereich im Wesentlichen von nur einem einzigen Laserpuls beaufschlagt wird.

Während der Bearbeitung kann die räumliche Lage des Fokus auf der Oberfläche des Bereichs konstant geregelt werden. Dies erfolgt, wie weiter unter noch näher ausgeführt werden wird, durch eine Autofokus-Einheit.

Gemäß eines weiteren Beispiels wird vor Aufbringen des Photosensitizers der zu bearbeitende Bereich dadurch ermittelt, dass auf das Gewebe ein Marker aufgebracht wird, welcher im Kontakt mit einer bestimmten Gewebeart, insbesondere geschädigtem Gewebe, eine charakteristische Färbung annimmt. Der Marker kann dabei ebenfalls durch einen Photosensitizer gegeben sein, der somit einen Diagnose-Photosensitizer darstellt, während der für die Ablation verwendete Photosensitizer als Ablations-Photosensitizer bezeichnet werden kann. Der Marker kann jedoch ebenso durch einen anderen handelsüblichen Marker gebildet sein, der keine Photosensitizer-Eigenschaften aufweist. Der Ablations-Photosensitizer ist dagegen vorzugsweise derart, dass er keine Marker-Eigenschaften aufweist, d.h. nichtfärbend (non-staining) bei Kontaktierung mit verschiedenen Gewebearten wirkt.

Gemäss eines weiteren Beispiels kann auch ein Photosensitizer verwendet werden, der zunächst als Marker eingesetzt wird und anschliessend für die Ablation verwendet wird. Dies könnte die Behandlung vereinfachen, indem nur diejenigen Bereiche des Photosensitizers, die einen Farbumschlag zeigen, mit dem Laser für die anschliessende Ablation bestrahlt werden.

Gemäß eines weiteren Beispiels wird der zu bearbeitende Bereich ohne Verwendung eines Markers ermittelt, indem nämlich das Vorhandensein eines in dem Gewebe erzeugten Signals und gegebenenfalls dessen Signalstärke ermittelt werden. Das Signal kann dabei die zweite oder eine höhere Harmonische einer auf das Gewebe eingestrahlten elektromagnetischen Strahlung sein. Die elektromagnetische Strahlung kann diejenige eines Diagnose-Laserstrahls sein, welcher eine Energiedichte auf der Oberfläche des Gewebes aufweist, welche kleiner ist als die Energiedichte, welche zur Bearbeitung des Gewebes erforderlich ist. Der Bearbeitungs-Laserstrahl und der Diagnose-Laserstrahl können von ein und derselben Laserstrahlquelle erzeugt werden. Insbesondere für die Unterscheidung zwischen nicht-geschädigtem Zahnmaterial und kariösem Zahnmaterial kann durch den Diagnose-Laserstrahl mittels des LIBS-Verfahrens (Laser Induced Breakdown Spectroscopy) im Infrarot-Bereich eine Anregung des Gewebes durchgeführt werden, wobei ein zurückgestreutes Signal einer zweiten Harmonischen ein gesundes Gewebe (z.B. mineralisationsfähige Kollagenfasern) anzeigt und das Ausbleiben eines solchen Signals ein kariöses Zahnmaterial (d.h. irreversibel geschädigte = mineralisationsunfähige Kollagenstrukturen) anzeigt. Ein Bereich des Gewebes kann mit dem Diagnose-Laserstrahl gescannt werden und die Daten der rückgestreuten zweiten Harmonischen können detektiert und gespeichert werden und auf der Basis dieser Daten kann dann bestimmt werden, in welchen Abschnitten des Bereiches eine Bearbeitung bzw. Ablation durch den Bearbeitungs-Laserstrahl erfolgen soll.

Erfindungsgemäß wird ein Laserbearbeitungsgerät zur Bearbeitung von biologischem Gewebe angegeben, wobei das Laserbearbeitungsgerät eine Laserstrahlquelle zum Bereitstellen eines gepulsten Bearbeitungs-Laserstrahls, eine Laserstrahl-Auskoppeleinheit zur Auskopplung des Laserstrahls in Richtung auf einen zu bearbeitenden Bereich des Gewebes, und eine Ausgabeeinrichtung zur Ausgabe eines Photosensitizers in Richtung auf eine Umgebung des zu bearbeitenden Bereichs des Gewebes aufweist, wobei die Ausgabeeinrichtung mit der Laserstrahl-Auskoppeleinheit verbunden ist.

Die Wellenlänge der Laserpulse ist beispielsweise derart auf das Material des Photosensitizers abgestimmt, dass der Bearbeitungs-Laserstrahl durch eine Zwei-Photonen-Absorption in dem Photosensitizer mindestens teilweise absorbiert wird, wobei die Absorption in der Nähe eines Absorptions-Maximums des Photosensitizers liegt.

Alternativ ist die Wellenlänge der Laserpulse derart auf das Material des Photosensitizers abgestimmt, das der Bearbeitungs-Laserstrahl durch eine Ein-Photonen-Absorption in dem Photosensitizer mindestens teilweise absorbiert wird, wobei die Absorption in der Nähe eines Absorptions-Maximums des Photosensitizers liegt.

Die Laserpulse weisen beispielweise eine Halbwertsbreite in einem Bereich zwischen 1 ps und 100 ps auf, wobei dieser Bereich auch jeden inkrementellen Zwischenwert umfasst und das Inkrement 100 fs beträgt.

Die Energiedichte der Laserpulse auf der Oberfläche des zu bearbeitenden Gewebes liegt beispielsweise in einem Bereich zwischen 1,5 J/cm² und 7,5 J/cm².

Die Energie der Laserpulse liegt beispielsweise in einem Bereich unterhalb von 100 µJ, wobei insbesondere ein Fokus des Bearbeitungs-Laserstrahls auf einer Oberfläche des Gewebes auf einen Fokusdurchmesser in einem Bereich zwischen 10 µm und 100 µm eingestellt ist.

Gemäß einer Ausführungsform ist eine Wiederholrate der Laserpulse in einem Bereich zwischen 1 Hz und 1000 kHz eingestellt.

Das Laserbearbeitungsgerät ist als dentales Laserbearbeitungsgerät zur Ablation oder Abtragung von Zahnmaterial, insbesondere kariösem Zahnmaterial, ausgebildet.

Gemäß einer Ausführungsform umfasst das Laserbearbeitungsgerät ein mit der Laserstrahl-Auskoppeleinheit verbundenes Fixiermittel zur räumlichen Fixierung eines distalen Endes der Laserstrahl-Auskoppeleinheit in Bezug auf einen Abschnitt des zu bearbeitenden Gewebes.

Gemäß eines Beispiels umfasst das Laserhandstück einen Aufsatz, der nicht nur als Fixiermittel dient, sondern auch den Zahn derart ummantelt, dass zusammen mit einem integrierten Absaugkanalsystem verschiedene chemische Zusammensetzungen und/oder verschiedene Druckverhältnisse generierbar sind, wodurch sogar Quecksilber, etwa in der Form einer Amalgam-Füllung, ablatierbar wird.

Gemäß einer Ausführungsform umfasst das Laserbearbeitungsgerät ferner eine Strahlformungs-Einheit zur Formung eines im Wesentlichen rechteckförmigen Strahlprofils des gepulsten Bearbeitungs-Laserstrahls.

Das Laserbearbeitungsgerät umfasst eine Scan-Einheit zum Abrastern oder Scannen eines Bereichs des Gewebes mit dem Bearbeitungs-Laserstrahl oder dem Diagnose-Laserstrahl. Dabei kann die Scan-Einheit insbesondere derart ausgelegt sein, dass ein von dem Fokus des Bearbeitungs-Laserstrahls erfasster Teilbereich von genau einem Laserpuls beaufschlagt wird. Beispielsweise kann die Scan-Einheit derart ausgelegt sein, dass einander benachbarte und von je einem Laserpuls erfasste Teilbereiche einen räumlichen Überlapp miteinander haben, dessen Fläche kleiner als die Hälfte eines Teilbereichs ist.

Das Laserbearbeitungsgerät umfasst ferner eine Autofokus-Einheit zum Konstanthalten der räumlichen Lage des Fokus auf der Oberfläche des Gewebes.

Das Laserbearbeitungsgerät umfasst ferner eine Detektions-Einheit zur Detektion des Vorhandenseins eines in dem Gewebe oder in einer Umgebung davon erzeugten Signals und gegebenenfalls von dessen Signalstärke. Die Detektions-Einheit weist erfindungsgemäß einen optischen Sensor auf, welcher für die Erfassung einer zweiten oder höheren Harmonischen einer auf das Gewebe eingestrahlten elektromagnetischen Strahlung ausgelegt ist. Die elektromagnetische Strahlung kann diejenige eines Diagnose-Laserstrahls sein, welcher von ein und derselben Laserstrahlquelle wie der Bearbeitungs-Laserstrahl erzeugt wird, oder sie kann diejenige einer kontinuierlich emittierenden Lichtemissionsdiode (LED) sein.

Das Laserbearbeitungsgerät kann eine Kontroll-Einheit aufweisen, welche dafür ausgelegt ist, die Laserstrahlquelle auf einen Bearbeitungsmodus oder einen Diagnosemodus einzustellen, wobei im Bearbeitungsmodus der gepulste Bearbeitungs-Laserstrahl erzeugt wird und im Diagnosemodus ein Diagnose-Laserstrahl erzeugt wird, welcher eine Energiedichte auf der Oberfläche des Gewebes aufweist, welche kleiner ist als die Energiedichte, welche zur Bearbeitung des Gewebes erforderlich ist.

Gemäß einer Ausführungsform kann die Laserstrahl-Auskoppeleinheit in der Form eines Handstücks vorgesehen sein, wobei die Ausgabeeinrichtung zur Ausgabe des Photosensitizers mindestens teilweise innerhalb des Handstücks angeordnet sein kann, wobei insbesondere ein Ende einer Zuleitung mit einer Düse in dem Handstück angeordnet sein kann. Innerhalb des Handstücks können auch zusätzlich die Scan-Einheit und/oder die Autofokus-Einheit angeordnet sein.

Das Laserbearbeitungsgerät kann ferner eine weitere Ausgabeeinrichtung für die Ausgabe eines Markers umfassen, wobei die weitere Ausgabeeinrichtung insbesondere ebenfalls teilweise innerhalb des Handstücks angeordnet sein kann.

Im Folgenden wird die Erfindung in Form weiterer Ausführungsformen anhand der Zeichnungen näher erläutert. Es zeigen:
- Fig. 1: eine schematische Darstellung einer Ausführungsform eines Laserbearbeitungsgeräts;
- Fig. 2: eine schematische Darstellung einer weiteren Ausführungsform eines Laserbearbeitungsgeräts; und
- Fig. 3: eine schematische Darstellung einer Ausführungsform einer Laserstrahl-Auskoppeleinheit;
- Fig. 4: eine schematische Darstellung einer Ausführungsform einer Laserstrahl-Auskoppeleinheit;
- Fig. 5: eine schematische Darstellung einer Ausführungsform einer Laserstrahl-Auskoppeleinheit
- Fig. 6: eine schematische Darstellung einer Ausführungsform einer Laserstrahl-Auskoppeleinheit;
- Fig. 7: eine schematische Darstellung einer Ausführungsform eines Laserbearbeitungsgeräts;
- Fig. 8: ein Flussdiagramm einer Ausführungsform eines automatisierten kombinierten Diagnose- und Bearbeitungsverfahrens;
- Fig. 9: ein Flussdiagramm einer Ausführungsform eines automatisierten kombinierten Diagnose- und Bearbeitungsverfahrens.

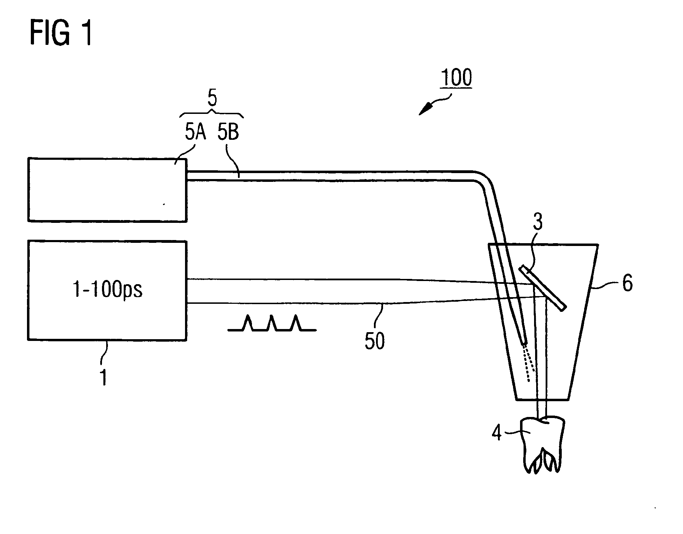

In der Fig. 1 ist eine Ausführungsform eines Laserbearbeitungsgeräts schematisch und mit nicht maßstabsgetreuen Größenverhältnissen dargestellt. Das Laserbearbeitungsgerät 100 ist im dargestellten Fall ein dentales Laserbearbeitungsgerät, mit welchem Zahnmaterial, insbesondere kariöses Zahnmaterial, bearbeitet bzw. abgetragen oder ablatiert werden kann. Das Laserbearbeitungsgerät kann jedoch auch ein anderes medizinisches Laserbearbeitungsgerät zur Bearbeitung einer anderen Art von biologischem Gewebe sein.

Das Laserbearbeitungsgerät 100 weist eine Laserstrahlquelle 1 auf, welche einen gepulsten Bearbeitungs-Laserstrahl 50 emittiert. Die Pulsdauer der Laserpulse liegt in einem Bereich zwischen 1 ps und 100 ps liegt. Der Bearbeitungs-Laserstrahl wird auf einen zu bearbeitenden Zahn 4 eines Patienten fokussiert. Gegebenenfalls kann der Bearbeitungs-Laserstrahl vorher durch eine optische Umlenkeinheit 3 wie einen Spiegel oder ein Umlenkprisma umgelenkt werden.

Es kann dabei vorgesehen sein, dass die Laserstrahlquelle 1 die Laserpulse solchermaßen generiert, dass diese eine Energie pro Puls in einem Bereich unterhalb von 100 µJ aufweisen. Die Fokussierung des Laserstrahls kann derart eingestellt werden, dass der Bearbeitungs-Laserstrahl 50 auf der Oberfläche des Zahns 4 einen Fokus mit einem Fokusdurchmesser in einem Bereich von 10 µm bis 100 µm aufweist.

Es kann weiter vorgesehen sein, dass die Laserstrahlquelle 1 die Laserpulse mit einer Repetitionsrate in einem Bereich von 1 Hz bis 1000 kHz emittiert.

Das Laserbearbeitungsgerät 100 weist ferner eine Ausgabeeinrichtung 5 zur Ausgabe eines Photosensitizers in Richtung auf eine Umgebung des zu bearbeitenden Bereichs des Zahns 4 auf. Die Ausgabeeinrichtung 5 kann wie dargestellt eine Vorratskammer 5A zur Bevorratung des Photosensitizers und eine damit verbundene Zuleitung 5B enthalten. Als Photosensitizer kann beispielsweise Erythrosin eingesetzt werden, welches durch Zwei-Photonen-Absorption der Laserstrahlung eines Nd:YAG-Lasers (1064 nm) oder durch Ein-Photonen-Absorption der frequenzverdoppelten Komponente des Nd:YAG-Lasers (532 nm) effizient angeregt werden kann. Als weitere Photosensitizer seien hier beispielhaft Methylen-Blau, Photofrin und metalloorganische Dendrimere genannt. Auch alle weiteren der einschlägigen Fachliteratur entnehmbaren Photosensitizer sowohl zukünftig noch zu entwickelnde Photosensitizer werden hiermit einbezogen, wobei stets die Laserwellenlänge auf das jeweilige Absorptionsmaximum des Photosensitizers oder zumindest eine Umgebung davon abzustimmen ist. Photosensitizer im hier verstandenen Sinne können auch biochemische Chromophore sein. Von dem Begriff Photosensitizer sollen definitionsgemäss auch solche Stoffe umfasst sein, die keine Photosensitizer im fachterminologischen Sinne sind, jedoch unter definierten physikalisch-chemischen Bedingungen die für Photosensitizer typischen Eigenschaften annehmen können. Dazu zählen beispielsweise im Prinzip beliebige Gase oder Gasgemische (Luft) oder Aerosole, wobei davon auszugehen ist, dass für alle diese Substanzen gilt, dass sie nur unter definierten physikalisch-chemischen Eigenschaften Photosensitizer-Eigenschaften zeigen.

Das Laserbearbeitungsgerät 100 weist ferner eine Laserstrahl-Auskoppeleinheit 6 zur Auskopplung des Laserstrahls 50 in Richtung auf einen zu bearbeitenden Bereich des Zahns 4 auf. Die Auskoppeleinheit 6 enthält in dem gezeigten Ausführungsbeispiel die Umlenk-Einheit 3 und ist des Weiteren mit der Zuleitung 5B der Ausgabeeinrichtung 5 verbunden, sodass an dem distalen Ende der Auskoppeleinheit 6 der Photosensitizer aus der Zuleitung 5B austritt und in der gewünschten Weise auf den gewünschten Bereich des Zahns 4 aufgebracht werden kann.

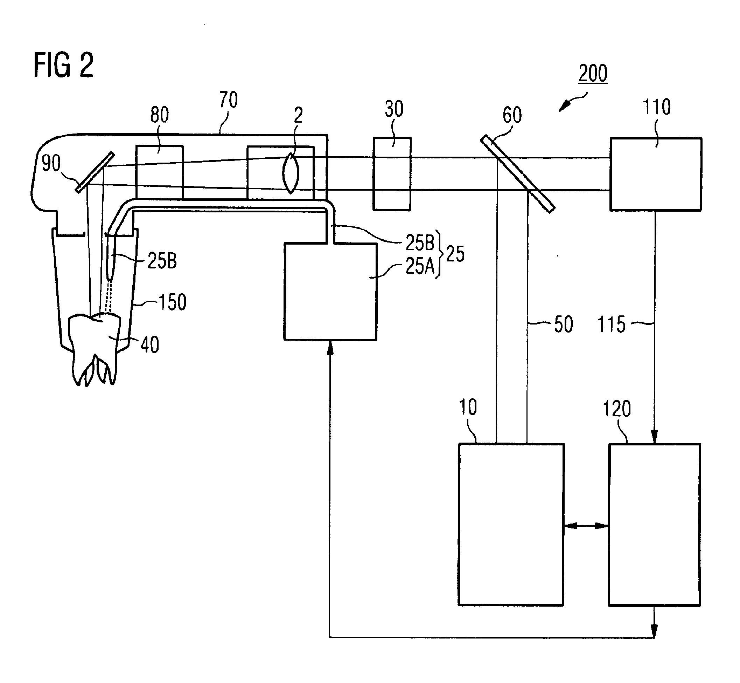

In der Fig. 2 ist eine weitere Ausführungsform eines Laserbearbeitungsgeräts schematisch und mit nicht maßstabgetreuen Größenverhältnissen dargestellt. Die in der Fig. 2 gezeigte Ausführungsform eines Laserbearbeitungsgeräts 200 umfasst eine Laserstrahlquelle 10, die einen gepulsten Bearbeitungs-Laserstrahl 50 emittiert. Die Laserstrahlquelle 10 ist in dem gezeigten Ausführungsbeispiel ein mit einem transienten oder einem regenerativen Verstärker gekoppelter Nd:YAG-Laser, welcher Laserpulse bei einer Wellenlänge von 1064 nm emittiert. Es kann hier auch eine andere Laserstrahlquelle wie beispielsweise ein Nd:YVO₄- oder ein Nd:GdVO₄-Las oder ein Laser, der einen anderen Nd-dotierten Laserkristall enthält, verwendet werden. Die Pulsdauer der Laserpulse beträgt 10 ps und die Wiederholrate der Laserpulse liegt in einem Bereich zwischen 1 kHz und 1000 kHz. Die Energie der Laserpulse beträgt 40 µJ. Bei einer Repetitionsrate von 100 kHz beträgt die mittlere Strahlungsleistung 4 W.

Als Laserstrahlquelle kann im Prinzip auch jeder andere Laser verwendet werden. Die Laserstrahlquelle kann beispielsweise auch durch einen Diodenlaser oder ein Diodenlaser-Array gegeben sein.

Der von der Laserstrahlquelle 10 emittierte Bearbeitungs-Laserstrahl 50 trifft in seinem Strahlenverlauf bei der in der Fig. 2 gezeigten Ausführungsform auf eine optische Umlenkeinheit 60, die jedoch nur für die Wellenlänge des Bearbeitungs-Laserstrahls 50 als Umlenkeinheit wirkt, sodass der Bearbeitungs-Laserstrahl 50 um ca. 90° umgelenkt wird.

Im Strahlengang des Bearbeitungs-Laserstrahls 50 befindet sich anschließend eine Strahlformungs-Einheit 30, mit welcher ein rechteckförmiges oder Tophat-Strahlprofil erzeugt wird.

Anschließend tritt der Bearbeitungs-Laserstrahl 50 in eine als ein Handstück ausgebildete Laserstrahl-Auskoppeleinheit 70 ein. Die Auskoppeleinheit 70 enthält in ihrem vorderen Abschnitt eine Linse 2, die Teil einer Autofokus-Einheit 20 ist. Die Autofokus-Einheit 20 bewirkt mit an sich bekannten Mitteln, dass der von der Linse 2 erzeugte Fokus stets innerhalb der Ebene der bearbeiteten Oberfläche des Zahns 40 liegt. Die Autofokus-Einheit 20 kann insbesondere mit einer optischen Sensoreinrichtung zusammenwirken, welche eine von der Oberfläche des Zahns zurückgeworfene Strahlung dahingehend auswertet, ob die Oberfläche noch im Fokus der Laserstrahlung liegt. Darauf hin wird ein Steuersignal an die Autofokus-Einheit 20 übermittelt, um eine geeignete Maßnahme zu ergreifen, so dass die Oberfläche des Zahns 40 wieder in den Fokus des Laserstrahls gelangt. Diese Maßnahme kann beispielsweise in einer Bewegung der Linse 2 vor oder zurück entlang des Ausbreitungsweges des Laserstrahls 50 bestehen. Dies kann durch einen schnellen Schrittmotor gewährleistet werden, der mit einem Schlitten verbunden ist, auf welchem die Linse 2 montiert ist. Es kann aber auch vorgesehen sein, dass die Linse 2 derart ausgestaltet ist, dass sich ihre Brechkraft verändern lässt.

Gemäß der Ausführungsform der Fig. 2 ist die Linse 2 derart angeordnet, dass sie auf der Oberfläche des Zahns 40 einen Fokus mit einem Fokusdurchmesser von 40 µm erzeugt. Mit dem weiter oben genannten Wert für die Pulsenergie eines Laserpulses ergibt sich somit eine Energiedichte von 3,18 J/cm², woraus sich wiederum eine Pulsspitzenintensität von 3,18 · 10¹¹ W/cm² ergibt. Dies entspricht einer Photonen-Flussdichte von 1,7 · 10³⁰ Photonen · cm⁻² · s⁻¹. Die elektrische Feldstärke des elektromagnetischen Wechselfeldes beträgt 1,55 · 10⁷ V/cm und die mittlere Oszillationsenergie der Elektronen im elektromagnetischen Wechselfeld beträgt 0,021 eV.

Es sei angemerkt, dass die Strahlformungseinheit 30 sich auch im Strahlengang hinter der Linse 2, also insbesondere auch im Handstück 70 befinden kann. Es kann auch vorgesehen sein, dass die Autofokuseinheit 20 und die Strahlformungseinheit 30, insbesondere die Linse 2 und die Strahlformungseinheit 30, zu einem gemeinsamen optischen Bauelement zusammengefasst werden.

In einem weiteren Abschnitt der Auskoppeleinheit 70 ist eine Scan-Einheit 80 angeordnet, mit welcher der Bearbeitungs-Laserstrahl 50 oder ein Diagnose-Laserstrahl in ebenfalls an sich bekannter Weise, beispielsweise mittels zweier sich gegenüberstehender rotierender Spiegel, über einen bestimmten Bereich der zu bearbeitenden Oberfläche des Zahns 40 gerastert oder gescannt werden kann. Mittels einer weiteren Umlenkeinheit 90, wie beispielsweise eines Umlenkprismas oder eines reflektierenden Spiegels wird dann der Bearbeitungs-Laserstrahl 50 oder ein Diagnose-Laserstrahl in Richtung auf den Zahn 40 umgelenkt.

In dieser Ausführungsform ist also die Scan-Einheit 80 im Handstück angeordnet. Es sind jedoch auch andere Ausführungsformen denkbar, in denen die Scan-Einheit im Strahlengang vor dem Handstück, d.h. insbesondere innerhalb eines Spiegelgelenkarms oder am Eingang eines Spiegelgelenkarms der optischen Einrichtung vor dem Handstück angeordnet ist.

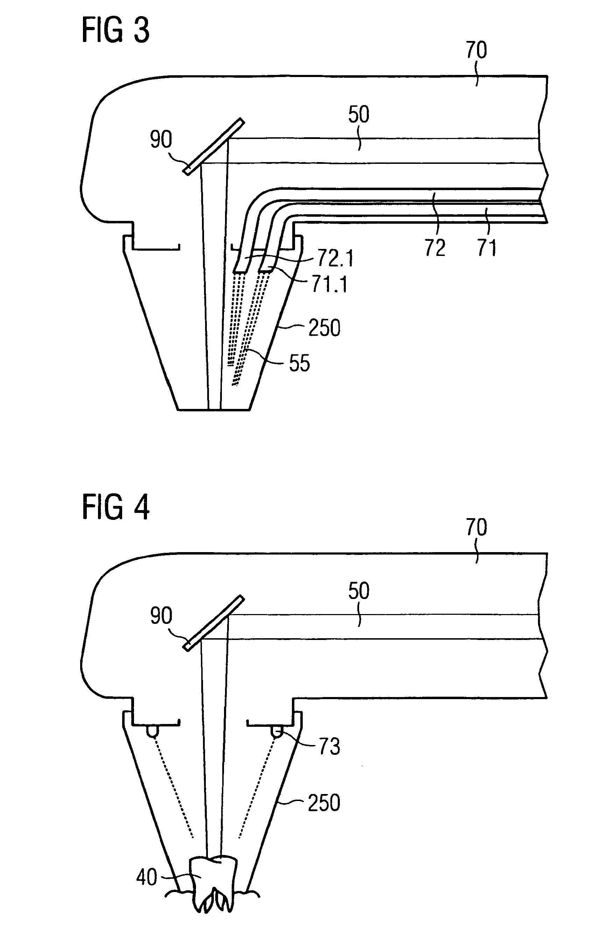

Die als ein Handstück ausgebildete Auskoppeleinheit 70 muss bei der Bearbeitung von dem behandelnden Arzt gehalten und auf den zu bearbeitenden Zahn gerichtet werden. Um diese Art von Handhabung zu erleichtern und insbesondere um die Position und Ausrichtung des distalen Endes der Auskoppeleinheit 70 in Bezug auf den zu bearbeitenden Zahn 40 konstant zu halten, ist ein trichterförmiges Fixierelement 150 an dem distalen Ende der Auskoppeleinheit 70 befestigt und kann während der Bearbeitung an dem Zahn 40 geeignet fixiert werden, wie in Verbindung mit den Ausführungsformen der Fig. 3 bis Fig.6 noch näher erläutert werden wird. Zuvor kann von dem behandelnden Arzt ein Kofferdam oder Spanngummi um den Zahn gelegt werden, um diesen von dem restlichen Mundraum zu trennen.

Das Laserbearbeitungsgerät 200 weist ferner eine Ausgabeeinrichtung 25 zur Ausgabe eines Photosensitizers in Richtung auf eine Umgebung des zu bearbeitenden Bereichs des Zahns 40 auf. Die Ausgabeeinrichtung 25 enthält eine Vorratskammer 25A und einen daran angeschlossenen Zuführungsschlauch 25B, welcher in die Auskoppeleinheit 70 einmündet und innerhalb der Auskoppeleinheit 70 bis in das Fixierelement 150 geführt wird.

Die in dem bearbeiteten Bereich der Oberfläche des Zahns 40 oder in einer Umgebung davon erzeugten optischen oder akustischen Signale können detektiert und zu Diagnosezwecken verwendet werden. Bezüglich optischer Signale wurde bereits erläutert, dass diese beispielsweise auf der zweiten (SHG, second harmonic generation) oder einer höheren Harmonischen von elektromagnetischer Strahlung beruhen, die auf das für eine Bearbeitung vorgesehene Zahnmaterial einwirkt. Das in der Fig. 2 gezeigte Ausführungsbeispiel soll im Folgenden anhand des Falls der Detektion eines SHG-Signals erläutert werden.

Bei dieser Art der Diagnose wird in der Laserstrahlquelle 10 ein Diagnose-Modus eingestellt, bei welchem ein Diagnose-Laserstrahl emittiert wird, dessen Energie oder Energiedichte unterhalb der Ablations- oder Plasma-Erzeugungsschwelle liegt, sodass mit diesem Laserstrahl keine Bearbeitung stattfinden kann. Mit dem Diagnose-Laserstrahl, welcher ebenso wie der Bearbeitungs-Laserstrahl gepulst ist, soll untersucht werden, ob ein zu bearbeitender Oberflächenbereich des Zahns gesundes oder kariöses Zahnmaterial enthält. Gesundes Zahnmaterial liefert ein höheres SHG-Signal als kariöses Material. Die solchermaßen an der Zahnoberfläche erzeugte frequenzverdoppelte Strahlung durchläuft in umgekehrter Richtung zumindest teilweise den Strahlengang des Bearbeitungs-Laserstrahls, wie er oben beschrieben worden ist, wird also von der Umlenkeinheit 90 umgelenkt, durchläuft die Scan-Einheit 80, die Autofokus-Einheit 20 mit der Linse 2 und trifft schließlich auf den Strahlteiler 60, der jedoch für die Wellenlänge des SHG-Signals durchlässig ist, sodass die frequenzverdoppelte Strahlung einer optischen Erfassungseinrichtung 110 zugeführt werden kann. Als optische Erfassungseinrichtung 110 kann ein einfacher Photodetektor vorgesehen sein, mit dem die Intensität der SHG-Strahlung gemessen wird. Es kann auch vorgesehen sein, dass als optische Erfassungseinrichtung 110 ein komplexeres System wie ein Spektrometer, eine CCD-Kamera oder ein CMOS-Bildsensor verwendet wird. Derartige optische Erfassungseinrichtungen können beispielsweise gleichzeitig dafür dienen, mit der Autofokus-Einheit 20, wie oben bereits angedeutet, in geeigneter Weise zusammenzuwirken.

Ebenso ist die Umlenkeinheit 90 so ausführbar, dass sie die an der Zahnoberfläche erzeugte frequenzverdoppelte Strahlung transmittiert und diese beispielsweise mit Hilfe einer Glasfaser, die sich hinter der Umlenkeinheit 90 befindet, zur optischen Erfassungseinrichtung 110 geleitet wird. Dadurch wird der Aufwand für den optischen Strahlengang zur Führung der frequenzverdoppelten Strahlung geringer, da die Optiken 80, 20, 60, 2 nicht für mehrere Wellenlänge ausgelegt und ggf. beschichtet sein müssen. Zur effizienteren Einkopplung des frequenzverdoppelten Lichts kann zwischen der Umlenkeinheit 90 und der Glasfaser eine Optik eingesetzt werden, die das frequenzverdoppelte Licht auf die Glasfaser fokussiert. Die Optik kann in Form einer Mikrooptik ausgeführt sein.

Die von der optischen Erfassungseinrichtung 110 ermittelten Werte für die SHG-Strahlung werden in ein Signal 115 umgewandelt und einer kombinierten Auswerte- und Steuereinheit 120 zugeführt, wobei es sich bei der Auswerte- und Steuereinheit 120 bei dieser Ausführungsform auch um ein Computersystem handeln kann. Es sind aber auch prinzipiell alle anderen Steuer- und Regelsysteme denkbar, wie z.B. SPS (speicherprogrammierbare Steuerungen), Mikro-Controller oder analoge Regelkreise.

Der Auswerte- und Steuereinheit 120 kann ein Signal von der Laserstrahlungsquelle 10 zugeführt werden, welches Daten über den Betriebszustand der Laserstrahlungsquelle 10 enthält. Die Auswerte- und Steuereinheit 120 gibt in Abhängigkeit von dem von der optischen Erfassungseinrichtung 110 übermittelten Signal 115 ein Steuersignal aus, welches der Laserstrahlungsquelle 10 zugeführt wird und die Laserstrahlungsquelle 10 beispielsweise von einem Ruhemodus in einen Bearbeitungsmodus schaltet.

Die in der Fig. 2 gezeigte Ausführungsform umfasst insbesondere eine Laserstrahlungsquelle 10, die zwischen den Betriebsarten "Aus" (Ruhemodus), "Diagnostik" und "Therapie" (Bearbeitung) umschalten kann. In dieser Ausführungsform emittiert die Laserstrahlquelle 10 sowohl den Bearbeitungs-Laserstrahl während dem "Therapie"-Modus als auch unter geänderten Bedingungen den Diagnose-Laserstrahl während dem "Diagnostik"-Modus. Der Unterschied zwischen dem Diagnostik- und dem Therapie-Modus ist im Wesentlichen die Pulsspitzenintensität in W/cm², die auf die Zahnoberfläche appliziert wird. Im Diagnose-Modus muss die Energiedichte sicher unterhalb der Ablations-Schwelle sein, während sie im Therapie-Modus darüber liegt.

In dem vorstehend beschriebenen Diagnosemodus kann beispielsweise ein bestimmter Oberflächenbereich des Zahns 40 mit dem Diagnose-Laserstrahl gescannt werden und das rückgestreute SHG-Signal empfangen und ausgewertet werden. Auf dieser Basis kann gewissermaßen der Oberflächenbereich kartiert werden, wobei ein zu bearbeitender oder zu ablatierender Oberflächenabschnitt identifiziert wird. Die Auswerte- und Steuereinheit 120 gibt nach Durchführung des Diagnosemodus ein Signal an die Ausgabeeinrichtung 25, woraufhin die Ausgabeeinrichtung 25 mittels des Zuleitungskanals 25B und der an dessen Ende montierten steuerbaren Düse den zu ablatierenden Bereich der Zahnoberfläche mit dem Photosensitizer beaufschlagt.

In der Fig. 3 ist eine Auskoppeleinheit 70 in der Form eines Handstücks in einem Querschnitt zur Illustration eines weiteren Ausführungsbeispiels für eine Diagnose dargestellt. In diesem Ausführungsbeispiel wird die Unterscheidung zwischen gesundem und geschädigtem Zahnmaterial nicht anhand eines SHG-Signals vorgenommen sondern anhand eines Markers, welcher in Kontakt mit geschädigtem Zahnmaterial eine charakteristische Färbung annimmt. Dieser Marker kann durch eine weitere Zuleitung 72, welche ebenfalls innerhalb des Handstücks verläuft, dem Zahn zugeführt werden. Nachdem auf solche Weise - vorzugsweise durch eine optische Aufnahme und anschließende Bildauswertung - festgestellt wurde, welche Bereiche einer Zahnoberfläche beispielsweise kariös sind, werden diese Bereiche mit dem Photosensitizer, der über die Zuleitung 71 dem Zahn zugeführt wird, beaufschlagt und können anschließend mit dem Bearbeitungs-Laserstrahl 50 ablatiert werden. In dieser Ausführungsform wird demgemäß kein Diagnose-Laserstrahl und keine Umschaltbarkeit der Laserstrahlquelle und ebenso keine SHG-Detektion benötigt. Mit beiden Zuleitungen 71 und 72 können steuer- und ausrichtbare Düsen 71.1 bzw. 72.1 verbunden sein, mit denen die Materialien zielgerichtet auf die Zahnoberfläche aufgebracht werden können.

Es sei angemerkt, dass die in der Fig.3 dargestellte Ausführungsform einer Laserstrahl-Auskoppeleinheit als eigenständige Komponente angesehen werden kann. Diese Laserstrahl-Auskoppeleinheit weist ein Handstück 70, eine Umlenkeinheit 90 zur Umlenkung eines Bearbeitungs-Laserstrahls 50 und/oder gegebenenfalls eines Diagnose-Laserstrahls, und einen Aufsatz 250 zur Fixierung des Handstücks 70 an einer Umgebung des zu bearbeitenden Gewebes auf, wobei das Handstück 70 so ausgebildet ist, dass durch eine in seinem Innenraum verlaufende Zuleitung 71 ein Photosensitizer und gegebenenfalls durch eine weitere Zuleitung 72 ein Marker auf einen zu bearbeitenden oder zu diagnostizierenden Gewebebereich auftragbar ist. Diese eigenständige Komponente kann auch mit jeder der in dieser Anmeldung beschriebenen anderen Ausführungsformen kombiniert werden und/oder mit jedem der in dieser Anmeldung beschriebenen Merkmale weitergebildet werden. Davon umfasst sind auch übergeordnete Einheiten wie ein Laserbearbeitungsgerät, welches eine Laserstrahl-Auskoppeleinheit wie vorstehend beschrieben enthält.

In der Fig. 4 ist eine Auskoppeleinheit 70 in der Form eines Handstücks in einem Querschnitt zur Illustration eines weiteren Ausführungsbeispiels dargestellt. Bei diesem Ausführungsbeispiel ist im Innenraum des Handstücks 70 mindestens eine Lichtemissionsdiode (LED) 73 angeordnet. Es können auch, wie in Fig.4 gezeigt, mehrere LEDs 73 vorhanden sein. Diese LEDs 73 können dem behandelnden Arzt dazu dienen, im noch nicht fixierten Zustand des Aufsatzes 250 den Mundraum auszuleuchten und ihm somit insbesondere zu ermöglichen, den Aufsatz 250 relativ zu dem zu behandelnden Zahn 40 optimal zu positionieren. Darüber hinaus können sie dazu dienen, einen auf der zu behandelnden Oberfläche des Zahns 40 aufgebrachten Marker anzuregen, so dass dieser an zu behandelnden, beispielsweise kariösen Stellen eine charakteristische Färbung annimmt. Das solchermassen von dem Marker erzeugte Bild kann mit derselben Optik erfasst werden, die für die Einkopplung des Bearbeitungs-Laserstrahls 50 verwendet wird, und kann ausgewertet werden. In einem nächsten Schritt kann dann auf der Basis dieser Auswertung der Photosensitizer auf den zu bearbeitenden bzw. zu ablatierenden Stellen aufgebracht werden. Die LEDs 73 können beispielsweise wie dargestellt auf einem horizontalen Endabschnitt des Handstücks 70 angeordnet und ihre räumliche Anordnung kann beispielsweise kreisförmig sein, so dass eine möglichst homogene und rotationssymmetrische Ausleuchtung erzielt werden kann. Die LEDs 73 sind mit elektrischen Zuleitungen (nicht dargestellt) verbunden, die innerhalb des Handstücks 70 geführt sind und durch die die LEDs 73 mit elektrischer Leistung versorgt werden. Die LEDs 73 können einfarbige, also quasi-monochromatische LEDs wie beispielsweise rotes Licht emittierende LEDs sein. Vorteilhaft könnte jedoch sein, Weisslicht-LEDs zu verwenden, da hiermit zum einen der Mundraum besser ausgeleuchtet wird und zum anderen unter Umständen damit eine grössere Auswahl an mit verschiedenen Wellenlängen anregbaren Markern zu Verfügung steht.

Es sei angemerkt, dass die in der Fig.4 dargestellte Ausführungsform einer Laserstrahl-Auskoppeleinheit als eigenständige Komponente angesehen werden kann. Diese Laserstrahl-Auskoppeleinheit weist ein Handstück 70, eine Umlenkeinheit 90 zur Umlenkung eines Bearbeitungs-Laserstrahls 50 und/oder gegebenenfalls eines Diagnose-Laserstrahls, einen Aufsatz 250 zur Fixierung des Handstücks 70 an einer Umgebung des zu bearbeitenden Gewebes und mindestens eine LED 73 zur Ausleuchtung und/oder zur Anregung eines Markers oder Photosensitizers eigenständige zers auf. Diese eigenständige Komponente kann auch mit jeder der in dieser Anmeldung beschriebenen anderen Ausführungsformen kombiniert werden und/oder mit jedem der in dieser Anmeldung beschriebenen Merkmale weitergebildet werden. Davon umfasst sind auch übergeordnete Einheiten wie ein Laserbearbeitungsgerät, welches eine Laserstrahl-Auskoppeleinheit wie vorstehend beschrieben enthält.

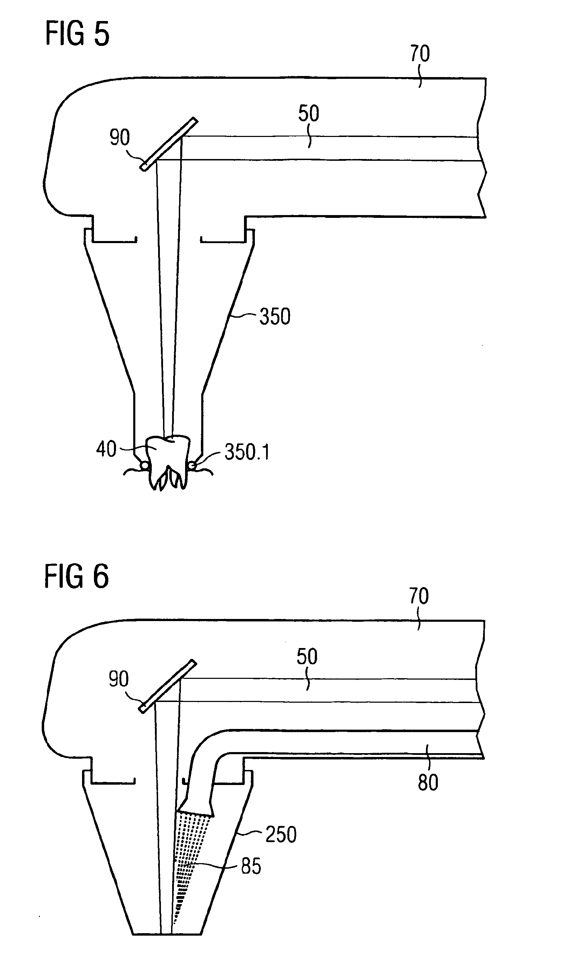

In der Fig.5 ist eine Auskoppeleinheit 70 in der Form eines Handstücks in einem Querschnitt zur Illustration eines weiteren Ausführungsbeispiels dargestellt. In diesem Ausführungsbeispiel ist das Handstück 70 mit einem Aufsatz 350 versehen, dessen Funktion darin besteht, das Handstück 70 nicht nur zu fixieren, sondern darüber hinaus den zu behandelnden Zahn 40 einzukapseln, wie hier durch eine an einem unteren Randabschnitt des Aufsatzes 350 angebrachte Dichtung 350.1 lediglich symbolhaft und nicht notwendigerweise technisch realistisch angedeutet werden soll. Ein Ziel einer derartigen gekapselten Fixierung besteht jedenfalls darin, eine unmittelbare Umgebung des zu behandelnden Zahns 40 möglichst weitgehend, d.h. insbesondere luft- und gasdicht gegenüber dem übrigen Mundraum abzuschliessen. Eine gekapselte Fixierung dieser Art schafft die Möglichkeit, die Zahnbehandlung auf verschiedenste Weise zu optimieren, wie in nachfolgenden Ausführungsbeispielen noch erörtert werden wird. Beispielsweise kann innerhalb des Handstücks 70 eine Absaugeinrichtung vorgesehen sein, durch die infolge der nach aussen dichtenden Fixierung ablatiertes Material besonders effizient und sicher abgesaugt werden kann. Des Weiteren kann eine kontrollierte Atmosphäre um den Zahn 40 geschaffen werden.

Es sei angemerkt, dass die in der Fig.5 dargestellte Ausführungsform einer Laserstrahl-Auskoppeleinheit als eigenständige Komponente angesehen werden kann. Diese Laserstrahl-Auskoppeleinheit weist ein Handstück 70, eine Umlenkeinheit 90 zur Umlenkung eines Bearbeitungs-Laserstrahls 50 und/oder gegebenenfalls eines Diagnose-Laserstrahls, einen Aufsatz 350 zur Fixierung des Handstücks 70 an einer Umgebung des zu bearbeitenden Gewebes auf, wobei der Aufsatz 350 so ausgebildet ist, dass er einen zu bearbeitenden Gewebebereich dicht umschliessen kann. Diese eigenständige Komponente kann auch mit jeder der in dieser Anmeldung beschriebenen anderen Ausführungsformen kombiniert werden und/oder mit jedem der in dieser Anmeldung beschriebenen Merkmale weitergebildet werden. Davon umfasst sind auch übergeordnete Einheiten wie ein Laserbearbeitungsgerät, welches eine Laserstrahl-Auskoppeleinheit wie vorstehend beschrieben enthält.

In der Fig.6 ist eine Auskoppeleinheit 70 in der Form eines Handstücks in einem Querschnitt zur Illustration eines weiteren Ausführungsbeispiels dargestellt. Bei diesem Ausführungsbeispiel ist auf das Handstück 70 ein Aufsatz 250 aufgesetzt und Handstück 70 und Aufsatz 250 sind derart ausgestaltet, dass darin ein Absaugkanal 80 aufgenommen ist, durch den bei der Bearbeitung von Gewebe ablatiertes Material effizient abgesaugt werden kann. Der Absaugkanal 80 ist mit einem Absaugkanalsystem (nicht dargestellt) verbunden und wird innerhalb des Handstücks 70 geführt und ragt mit seinem Öffnungsende derart in den Aufsatz 250 hinein, dass dieses auf die bearbeitete Stelle ausgerichtet ist und im Betrieb ablatiertes Material 85 absaugt. Es kann vorgesehen sein, dass das Ende des Absaugkanals 80 beweglich gehaltert ist und insbesondere in anwenderseitigen steuerbar und ausrichtbar ist, wobei auch der Abstand zu der bearbeiteten Stelle veränderbar sein kann.

Es sei angemerkt, dass die in der Fig.6 dargestellte Ausführungsform einer Laserstrahl-Auskoppeleinheit als eigenständige Komponente angesehen werden kann. Diese Laserstrahl-Auskoppeleinheit weist ein Handstück 70, eine Umlenkeinheit 90 zur Umlenkung eines Bearbeitungs-Laserstrahls 50 und/oder gegebenenfalls eines Diagnose-Laserstrahls, einen Aufsatz 250 zur Fixierung des Handstücks 70 an einer Umgebung des zu bearbeitenden Gewebes auf, wobei das Handstück 70 und der Aufsatz 350 so ausgebildet sind, dass in ihnen ein Absaugkanal 80 geführt ist, dessen Ende auf einen zu bearbeitenden Gewebebereich ausrichtbar ist. Diese eigenständige Komponente kann auch mit jeder der in dieser Anmeldung beschriebenen anderen Ausführungsformen kombiniert werden und mit jedem in dieser Anmeldung beschriebenen Merkmal weitergebildet werden. Davon umfasst sind auch übergeordnete Einheiten wie ein Laserbearbeitungsgerät, welches eine Laserstrahl-Auskoppeleinheit wie vorstehend beschrieben enthält. Insbesondere eine Kombination der in den Fig.5 und 6 beschriebenen Ausführungsformen, d.h. eine gekapselte, dichte Fixierung mit einem Absaugsystem bietet die Möglichkeit, dass auch potentiell toxische Bearbeitungsprozesse wie beispielsweise die Ablation von Amalgam-Füllungen durchgeführt werden können. Im letzteren Fall sorgt die Absaugung in Verbindung mit dem gasdichten Abschluss dafür, dass das ablatierte Material, im Wesentlichen elementares Quecksilber, sicher und weitgehend rückstandsfrei entfernt werden kann. Die Laserablation der Amalgam-Füllung könnte beispielsweise, wie in dieser Anmeldung beschrieben, mit der Unterstützung eines Photosensitizers durchgeführt werden. Mit der hier dargestellten Erfindung könnte somit eine Amalgam-Entfernung mittels Laser unter Einhaltung gesetzlich vorgeschriebener MAK-Werte (Maximale Arbeitsplatz-Konzentration) für Quecksilberdämpfe durchgeführt werden.

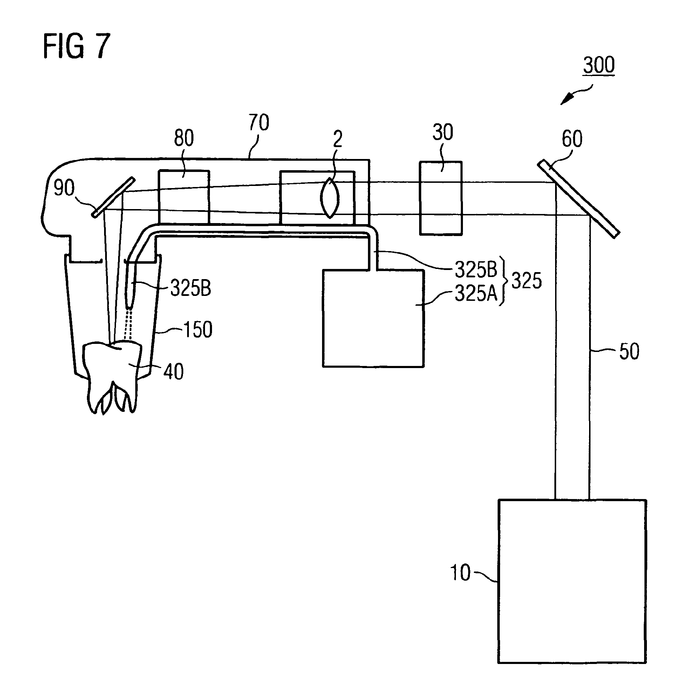

In der Fig.7 ist eine weitere Ausführungsform eines Laserbearbeitungsgeräts schematisch und mit nicht maßstabgetreuen Größenverhältnissen dargestellt. Die in der Fig.7 gezeigte Ausführungsform eines Laserbearbeitungsgeräts 300 entspricht in wesentlichen Teilen der in der Fig.2 dargestellten Ausführungsform, wobei bezüglich der Elemente mit gleichen Bezugszeichen auf die Ausführungen zu Fig.2 verwiesen wird. Das Laserbearbeitungsgerät 300 weist im Unterscheid zu Fig.2 eine Generatoreinheit 325 auf, die einen Generator 325A aufweist, die mit dem Handstück 70 durch eine Leitung 325B verbunden ist. Die Leitung 325B wird durch das Handstück bis in den Aufsatz 150 geführt und weist an ihrem Ende eine Öffnung auf, die in Richtung auf den zu bearbeitenden Zahn ausgerichtet ist. Die in dieser Allgemeinheit dargestellte Generatoreinheit 325 kann verschiedene Funktionalitäten beinhalten. Sie dient in erster Linie dazu, in einer Umgebung des zu bearbeitenden Zahns 40 eine bestimmte Atmosphäre zu erzeugen. In einer einfachen Variante kann mit der Generatoreinheit 325 ein Vakuum erzeugt werden, wobei die Generatoreinheit 325 eine Vakuumpumpe aufweist. Der Aufsatz 150 kann in diesem Fall wie der Aufsatz 350 in der Ausführungsform der Fig.5, also als kapselnde Fixierung ausgebildet und zusätzlich kann der Aufsatz 150 auch noch - falls gewünscht oder erforderlich - gegen das Handstück 70 abgedichtet sein, wobei zwischen dem Handstück 70 und dem Aufsatz 150 ein für den Laserstrahl 50 durchlässiges Fenster angeordnet ist. In einer etwas weniger aufwendigen Form ist hingegen keine oder zumindest keine vollständige Abdichtung zwischen Handstück 70 und Aufsatz 150 vorgesehen, wenn z.B. nur ein Unterdruck oberhalb des Zahns 40 erzeugt werden soll. Die Generatoreinheit 325 kann auch dafür ausgelegt sein, einen Überdruck zu erzeugen. Des Weiteren kann die Generatoreinheit 325 dafür ausgelegt sein, eine bestimmte Gas-Atmosphäre in der Umgebung des Zahns 40 zu erzeugen, etwa durch Bereitstellung eines Gases wie O₂, N₂, H₂O (Wasserdampf) oder eines Edelgases. Insbesondere für die Ablation von Amalgam-Füllungen kann dies in vorteilhafter Weise dazu benutzt werden, das ablatierte Quecksilber in bestimmter Weise zu binden und aus der Umgebung des Zahns 40 zu entfernen. Die Generatoreinheit 325 kann auch dafür ausgelegt sein, eine Kühlung für den Zahn 40 zu ermöglichen, etwa indem ein Kühlmedium, im einfachsten Fall Kühlungsluft erzeugt und auf den ablatierten Oberflächenbereich geblasen wird. Die Generatoreinheit 325 kann auch als ein Aerosol-Generator ausgelegt sein, indem sie beispielsweise ein Gas erzeugt, in welchem Partikel wie mikroskopische (Nano-) oder makroskopische Partikel dispergiert sind und die bestimmte Funktionen im Zusammenhang mit dem Ablationsvorgang übernehmen können. Die Partikel können auch ihrerseits eine Kühlungsfunktion übernehmen. Im übrigen können die Steuereinheit 120 und der Detektor 110 der Ausführungsform der Fig.2 hier ebenfalls vorhanden sein, wobei die Steuereinheit 120 überdies mit der Generatoreinheit 325 verbunden sein und diese steuern kann.

Das in der Fig.7 dargestellte Beispiel eines Laserbearbeitungsgeräts weist eine Laserstrahlquelle 10 zum Bereitstellen eines Bearbeitungs-Laserstrahls 50, eine Laserstrahl-Auskoppeleinheit 70 zur Auskopplung des Laserstrahls 50 in Richtung auf einen zu bearbeitenden Bereich eines Gewebes und eine Generatoreinheit 325 zur Erzeugung oder Bereitstellung einer Atmosphäre in einer Umgebung des zu bearbeitenden Gewebes auf.

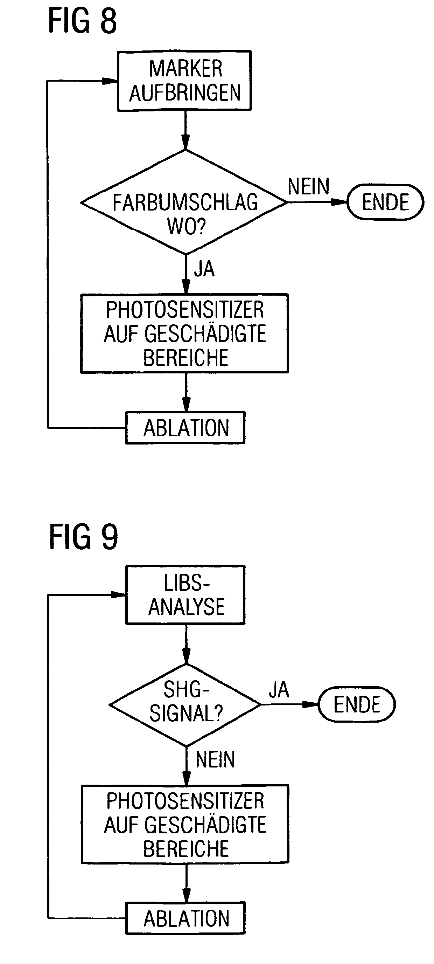

In der Fig.8 ist ein Flussdiagramm für ein Beispiel eines automatisierten kombinierten Ablations- und Diagnoseverfahrens bei Verwendung eines Markers dargestellt. In einem Schritt s1 wird ein Marker aufgebracht. In einem Schritt s2 wird festgestellt, ob sich ein Farbumschlag ergeben hat, der geschädigtes Gewebe anzeigt. Wenn kein Farbumschlag feststellbar ist, ist das Verfahren beendet. Die Feststellung des Farbumschlags kann ortsaufgelöst erfolgen, indem die Oberfläche auf einen Detektor wie ein CCD- oder CMOS-Element abgebildet wird und die Bereiche mit Farbumschlag durch eine Bilderkennung erfasst und elektronisch gespeichert werden. Anschliessend wird der Marker entfernt und in einem Schritt s4 wird ein Photosensitizer auf die als geschädigt festgestellten Bereiche aufgebracht. In einem nächsten Schritt s5 wird die Ablation mit dem Laserstrahl durchgeführt, wobei die Bearbeitungsparameter wie Dauer der Bearbeitung, Leistung und dergleichen vorher vom Benutzer eingestellt werden. Anschliessend wird erneut im Schritt s1 der Marker aufgebracht und im Schritt s2 festgestellt, ob noch geschädigte Bereiche vorhanden sind.

In der Fig.9 ist ein Flussdiagramm für ein Beispiel eines automatisierten kombinierten Ablations- und Diagnoseverfahrens bei Verwendung des LIBS-Verfahrens dargestellt. In einem Schritt s1 wird ein Bereich mit einem Diagnose-Laserstrahl gescannt und simultan die Detektion eines SHG-Signals detektiert, wie im Zusammenhang mit Fig.2 beschrieben. In einem Schritt s2 wird festgestellt, welche Bereiche ein SHG-Signal zurückgestreut haben und somit als gesunde Bereiche anzusehen sind. Wenn ganzflächig ein SHG-Signal empfangen wurde, ist das Verfahren beendet. Die Feststellung der gesunden Bereiche kann demgemäss auch hier durch den Detektor ortsaufgelöst erfolgen und die dazu komplementären Bereiche können als geschädigte Bereiche elektronisch gespeichert werden. Anschliessend wird in einem Schritt s4 ein Photosensitizer auf die als geschädigt festgestellten Bereiche aufgebracht. In einem nächsten Schritt s5 wird die Ablation mit dem Laserstrahl durchgeführt, wobei die Bearbeitungsparameter wie Dauer der Bearbeitung, Leistung und dergleichen vorher vom Benutzer eingestellt werden. Anschliessend wird erneut im Schritt s1 eine LIBS-Analyse durchgeführt und im Schritt s2 festgestellt, ob noch geschädigte Bereiche vorhanden sind.

Die in den Fig.8 und 9 dargestellten kombinierten Bearbeitungs- und Diagnoseverfahren weisen folgende Schritte auf, sind jedoch nicht Teil der Erfindung: Feststellen geschädigter Bereiche mittels Marker oder LIBS, Aufbringen eines Photosensitizers auf die geschädigten Bereiche, Ablation der geschädigten Bereiche mittels eines Laserstrahls, erneutes Feststellen einer Schädigung und gegebenenfalls wiederholtes Aufbringen eines Photosensitizers und anschliessende Ablation, bis keine Schädigung mehr feststellbar ist.

## Patentansprüche

1. Dentales Laserablationsgerät zur Ablation von Zahnmaterial, insbesondere kariösem Zahnmaterial, umfassend:
eine Laserstrahlquelle (1; 10) zum Bereitstellen eines gepulsten Ablations-Laserstrahls (50);
eine Laserstrahl-Auskoppeleinheit (6; 70) zur Auskopplung des Laserstrahls (50) in Richtung auf einen zu ablatierenden Bereich eines Zahns;
eine Ausgabeeinrichtung (5; 25) zur Ausgabe eines Photosensitizers in Richtung auf einen zu ablatierenden Bereich des Zahnmaterials, wobei die Ausgabeeinrichtung (5; 25) mit der Laserstrahl-Auskoppeleinheit (6; 70) verbunden ist;
eine Scan-Einheit (80) zum Abrastern oder Scannen eines Bereichs des Zahnmaterials mit dem Ablations-Laserstrahl (50), wobei die Scan-Einheit (80) derart ausgelegt ist, dass ein von dem Fokus des Ablations-Laserstrahls (50) erfasster Teilbereich von genau einem Laserpuls beaufschlagt wird;
eine Autofokus-Einheit (20) zum Konstanthalten der räumlichen Lage des Fokus auf der Oberfläche des Zahnmaterials;
eine Detektions-Einheit (110) zur Detektion des Vorhandenseins eines in dem Zahnmaterial oder in einer Umgebung davon erzeugten Signals und gegebenenfalls von dessen Signalstärke, wobei die Detektions-Einheit (110) einen optischen Sensor aufweist, **gekennzeichnet dadurch, dass** der optische Sensor für die Erfassung einer zweiten oder höheren Harmonischen einer auf das Zahnmaterial eingestrahlten elektromagnetischen Strahlung ausgelegt ist.

2. Laserablationsgerät nach Anspruch 1, bei welchem eine Wiederholrate der Laserpulse in einem Bereich zwischen 1 Hz und 1000 kHz eingestellt ist.

3. Laserablationsgerät nach einem der Ansprüche 1 oder 2, ferner umfassend:
ein mit der Laserstrahl-Auskoppeleinheit (6; 70) verbundenes Fixiermittel (150) zur räumlichen Fixierung eines distalen Endes der Laserstrahl-Auskoppeleinheit (6; 70) in Bezug auf einen Abschnitt des zu ablatierenden Zahnmaterials.

4. Laserablationsgerät nach einem der Ansprüche 1 bis 3, ferner umfassend:
eine Strahlformungs-Einheit (30) zur Formung eines im Wesentlichen rechteckförmigen Strahlprofil des gepulsten Ablations-Laserstrahls (50).

5. Laserablationsgerät nach einem der vorhergehenden Ansprüche, bei welchem
die Scan-Einheit (80) derart ausgelegt ist, dass einander benachbarte und von je einem Laserpuls erfasste Teilbereiche einen räumlichen Überlapp miteinander haben, dessen Fläche kleiner als die Hälfte eines Teilbereichs ist.

6. Laserablationsgerät nach einem der vorhergehenden Ansprüche, bei welchem
die Laserstrahl-Auskoppeleinheit (70) als ein Handstück ausgebildet ist, in welchem die Ausgabeeinrichtung (25) zur Ausgabe des Photosensitizers mindestens zum Teil enthalten ist.

7. Laserablationsgerät nach Anspruch 6, bei welchem in dem Handstück die Scan-Einheit (80) und/oder die Autofokus-Einheit (20) enthalten ist/sind.

## Claims

1. Dental laser ablation instrument for ablating tooth material, more particularly carious tooth material, comprising:
a laser beam source (1; 10) for providing a pulsed ablation laser beam (50);
a laser beam decoupling unit (6; 70) for decoupling the laser beam (50) in the direction of a region of a tooth to be ablated;
an emission apparatus (5; 25) for emitting a photosensitizer in the direction of a region of the tooth material to be ablated, wherein the emission apparatus (5; 25) is connected to the laser beam decoupling unit (6; 70);
a scan unit (80) for scanning a region of the tooth material with the ablation laser beam (50), wherein the scan unit (80) is configured in such a way that a portion captured by the focus of the ablation laser beam (50) is impinged on by precisely one laser pulse;
an autofocus unit (20) for keeping constant the spatial position of the focus on the surface of the tooth material;
a detection unit (110) for detecting the presence of a signal generated in the tooth material or in the surroundings thereof and for optionally detecting the signal strength thereof, wherein the detection unit (110) has an optical sensor, **characterized in that** the optical sensor is configured for capturing a second or higher harmonic of electromagnetic radiation radiated onto the tooth material.

2. Laser ablation instrument according to Claim 1, wherein a repetition rate of the laser pulses is set in a region between 1 Hz and 1000 kHz.

3. Laser ablation instrument according to either of Claims 1 or 2, furthermore comprising:
a fixation means (150), connected to the laser beam decoupling unit (6; 70), for spatially fixing a distal end of the laser beam decoupling unit (6; 70) with respect to a section of the tooth material to be ablated.

4. Laser ablation instrument according to one of Claims 1 to 3, furthermore comprising:
a beam forming unit (30) for forming a substantially rectangular beam profile of the pulsed ablation laser beam (50).

5. Laser ablation instrument according to one of the preceding claims, wherein
the scan unit (80) is configured in such a way that portions adjacent to one another and respectively captured by a laser pulse have a spatial overlap with one another, the area of which spatial overlap is smaller than half of a portion.

6. Laser ablation instrument according to one of the preceding claims, wherein
the laser beam decoupling unit (70) is embodied as a handpiece, in which the emission apparatus (25) for emitting the photosensitizer is at least partly contained.

7. Laser ablation instrument according to Claim 6,
wherein
the scan unit (80) and/or the autofocus unit (20) is or are contained within the handpiece.

## Revendications

1. Appareil dentaire d'ablation au laser destiné à l'ablation de matière dentaire, notamment de matière dentaire cariée, comportant :
une source de faisceau laser (1 ; 10) mise à la disposition d'un faisceau laser d'ablation pulsé (50) ;
une unité d'extraction du faisceau laser (6 ; 70) pour l'extraction du faisceau laser (50) en direction d'une zone d'une dent devant subir l'ablation ;
un équipement de distribution (5 ; 25) pour la distribution d'un photo-sensibilisateur en direction d'une zone de matière dentaire devant subir l'ablation, l'équipement de distribution (5 ; 25) étant relié à l'unité d'extraction du faisceau laser (6 ; 70) ;
une unité de balayage (80) pour décrire une trame ou pour balayer une zone de la matière dentaire au moyen du faisceau laser d'ablation (50), l'unité de balayage (80) étant développée de telle sorte qu'une zone partielle touchée par le point focal du faisceau laser d'ablation (50) est touchée par une impulsion laser exactement ;
une unité d'autofocus (20) pour le maintien constant de la position spatiale du point focal sur la surface de la matière dentaire ;
une unité de détection (110) pour la détection de la présence d'un signal généré dans la matière dentaire ou à proximité de celle-ci, et éventuellement de la force de ce signal, l'unité de détection (110) comportant un capteur optique, **caractérisé en ce que** le capteur optique pour l'enregistrement d'une deuxième harmonique ou d'une harmonique plus élevée est exposé à un rayonnement électromagnétique irradiant la matière dentaire.

2. Appareil d'ablation au laser selon la revendication 1, dans lequel une fréquence de répétition des impulsions laser est réglée dans une plage entre 1 Hz et 1000 kHz.

3. Appareil d'ablation au laser selon une des revendications 1 ou 2, comportant de plus :
un moyen de fixation (150) relié à l'unité d'extraction du faisceau laser (6 ; 70) pour la fixation dans l'espace d'une extrémité distale de l'unité d'extraction du faisceau laser (6 ; 70) par rapport à une section de la matière dentaire devant subir l'ablation.

4. Appareil d'ablation au laser selon une des revendications 1 à 3, comportant de plus :
une unité de mise en forme du faisceau (30) pour la mise en forme d'un profil de faisceau de forme essentiellement rectangulaire du faisceau laser d'ablation pulsé (50).

5. Appareil d'ablation au laser selon une des revendications précédentes, dans lequel :
l'unité de balayage (80) est conçue de telle sorte que des zones partielles mutuellement voisines et enregistrées chacune par une impulsion laser présentent une superposition dans l'espace dont la surface est inférieure à la moitié d'une zone partielle.

6. Appareil d'ablation au laser selon une des revendications précédentes, dans lequel :
l'unité d'extraction du faisceau laser (70) est développée sous la forme d'une pièce manuelle contenant au moins partiellement l'équipement de distribution (25) pour la distribution du photo-sensibilisateur.

7. Appareil d'ablation au laser selon la revendication 6, dans lequel la pièce manuelle contient l'unité de balayage (80) et/ou l'unité d'autofocus (20).
